# FASCICULE DE BREVET EUROPEEN

(11) **EP 0 960 642 B1**
(45) Date de publication et mention de la délivrance du brevet: **05.07.2006**
(21) Numéro de dépôt: 99401138.5
(22) Date de dépôt: 07.05.1999
(51) Int. Cl.: B01D 15/02, C07C 15/08

(54) **Unité de séparation comprenant un dispositif de régulation de débit**
Trennvorrichtung enthaltend Vorrichtung zur Regelung des Volumenstroms von wenigstens einem Fluid
Separation unit comprising a device for controlling the flow of at least one fluid

(30) Priorité: 29.05.1998 FR 9806788
(43) Date de publication de la demande: 01.12.1999
(73) Titulaire: Institut Français du Pétrole, 92852 Rueil-Malmaison Cedex (FR)
(72) Inventeur: Hotier, Gérard, 69630 Chaponost (FR); Le Corre, Albert, 78800 Houilles (FR); Humeau, Dominique, 38200 Vienne (FR); Renard, Pierre, 78860 Saint Nom la Breteche (FR)

(56) Documents cités:
- EP-A- 0 415 822
- FR-A- 2 743 002
- US-A- 5 156 736

## Description

L'invention concerne un dispositif et un procédé de régulation indirecte de débit d'au moins un fluide, un effluent par exemple, dans une zone chromatographique contenant une phase fixe, adsorbant ou tamis moléculaire par exemple. Plus particulièrement elle permet d'obtenir des pressions à l'intérieur d'une plage de pressions prédéterminée en amont et en aval d'une ou de préférence des vannes de régulation dans une zone d'adsorption à lit mobile simulé, à contre-courant ou à co-courant.

Elle s'applique, notamment, à la séparation d'au moins un isomère de xylène et en particulier du paraxylène à partir de charges d'hydrocarbures en contenant.

L'art antérieur est illustré par les brevets EP-A-415.822, US-5,156,736 et FR-A- 2.743.002 qui décrit la mise en oeuvre la plus performante.

Un lit mobile simulé comporte au moins trois zones chromatographiques, avantageusement quatre ou cinq, chacune de ces zones étant constituée par au moins une colonne ou un tronçon de colonne. Au moins un point entre deux zones sert à l'injection d'une charge à fractionner et au moins un point entre deux zones sert à l'injection d'un éluant ou désorbant ou solvant. De plus, le lit mobile simulé comporte au moins un point de soutirage d'un extrait entre le point d'injection d'éluant et le point d'injection de la charge situé en aval dans le sens de circulation de le désorbant et au moins un point de soutirage d'un raffinat entre chaque point d'injection du mélange et le point d'injection d'éluant situé en aval dans le sens de la circulation de le désorbant.

L'ensemble des colonnes ou tronçons forme au moins une boucle fermée contenant au moins une pompe de recyclage, entre deux tronçons, régulée en débit (entre le premier et le dernier tronçon par exemple).

On décale, généralement, dans le temps dans un même sens (aval ou amont) les points d'injection et de soutirage d'au moins un tronçon ou colonne.

Il est primordial de respecter les débits d'effluents qui circulent d'une zone à l'autre et qui doivent rester sensiblement constants dans une zone donnée. En effet, une faible variation de débit, même dans une seule zone peut avoir une très grande influence sur le résultat de la séparation.

On considère, par exemple, le cas d'un lit mobile simulé, à contre-courant comportant quatre zones avec une pompe de recyclage et deux flux entrant, le désorbant et la charge, et deux flux sortant, l'extrait et le raffinat.

La zone 1 est située entre le désorbant et l'extrait, la zone 2 est située entre l'extrait et la charge, la zone 3 est située entre la charge et le raffinat et la zone 4 entre le raffinat et le désorbant. Les débits dans les différentes zones sont les suivants lorsque la pompe est en zone 1 par exemple:
Débit en zone 1: débit de la pompe.
Débit en zone 2: débit en zone 1 - débit de l'extrait
Débit en zone 3: débit en zone 2 + débit de charge
Débit en zone 4: débit en zone 3 - débit de raffinat.

Toutes les erreurs sur les débits des flux entrant ou sortant se répercutent donc sur le débit de recyclage et doivent donc être contrôlées avec précision.

Chaque fois qu'un des flux entrant ou sortant de la boucle passe de part et d'autre de la pompe de recyclage, par exemple d'une position immédiatement antérieure à une position immédiatement postérieure à la pompe de recyclage lorsqu'on opère à contre-courant simulé, deux difficultés apparaissent au point de vue de la régularité des débits:
- la première difficulté concerne la pompe de recyclage, quand elle change de zone. Il est très important que son débit soit quasiment instantanément modifié et que le nouveau débit, celui de la nouvelle zone où elle se trouve, soit régulé avec précision et de manière stable sans que le passage d'un débit à l'autre se fasse de manière trop lente (cas d'une régulation amortie) ou avec des fluctuations autour de la nouvelle valeur (cas d'une régulation ayant des actions rapides).
   Ce premier problème technique est en relation avec le changement de zones. Il est, en effet, très important que le débit passe d'une première valeur à une autre valeur désirée instantanément. On a, par exemple, constaté qu'avec une variation du débit de recyclage de 0,6 %, on observait une variation de 4,2 % sur la pureté.
   Il s'avère, en effet, qu'un régulateur de débit régulant un débit de manière stable, auquel on délivre une nouvelle consigne, met un certain temps à réguler à la nouvelle valeur. Le passage d'un débit à l'autre devant être rapide, il faut que le gain du régulateur soit important. Dans ce cas, la régulation n'est pas très stable. On a donc le choix entre une régulation rapide mais fluctuante ou une régulation fine et stable mais avec de l'inertie. Ces deux solutions ne sont pas acceptables pour réguler un lit mobile simulé dont l'objectif est d'atteindre des hautes puretés.
- la deuxième difficulté concerne le débit du flux entrant, ou sortant, de l'unité. En effet, le débit de ce flux doit être maintenu constant, et avec une très bonne précision, alors que son point d'injection, ou de soutirage, passe d'une pression basse, la pression d'aspiration de la pompe, à une pression élevée, la pression de refoulement de la pompe (la différence de pression correspond à la perte de charge dans l'ensemble des colonnes ou tronçon de colonne).

Il est très important de bien résoudre ces problèmes pour obtenir de bons résultats de séparation.

La solution proposée, par l'art antérieur, consiste à ne pas laisser le régulateur agir tout seul mais, en utilisant un automate, un ordinateur, ou tout autre moyen pouvant agir sur le régulateur, de manière à imposer audit régulateur de stopper temporairement sa régulation, de lui imposer simultanément de modifier son action (prépositionnement de l'action), de manière à ce que la nouvelle action imposée, tel qu'un pourcentage d'ouverture de vanne, une fréquence de courant pour un moteur, un voltage, etc., corresponde aux nouvelles conditions assurant une bonne régulation du débit considéré, et ensuite, mais en fait presque immédiatement, à remettre en action le régulateur:
- dans le cas de la pompe de recyclage pour changer un débit d'une zone à l'autre,
- dans le cas d'un effluent pour maintenir un débit en présence d'une forte variation des conditions de pression.

On obtient, ainsi, les bons débits sans oscillations et pratiquement instantanément. L'ensemble des opérations décrites dure entre 1/100 et 10 secondes et le plus souvent entre 1/10 et 5 secondes suivant les cas.
Les exemples du texte de l'art antérieur s'appliquent à une unité pilote comportant par exemple 24 lits et une unique pompe de recirculation.
Lorsque le procédé de régulation des débits selon l'art antérieur (FR 95/15526 ) est appliqué à l'unité décrite dans les figures 1 A, 1 B et 2, on constate que des perturbations résiduelles subsistent en particulier sur le débit de raffinat (voir figures 3A, 3B, 3D, 3C).

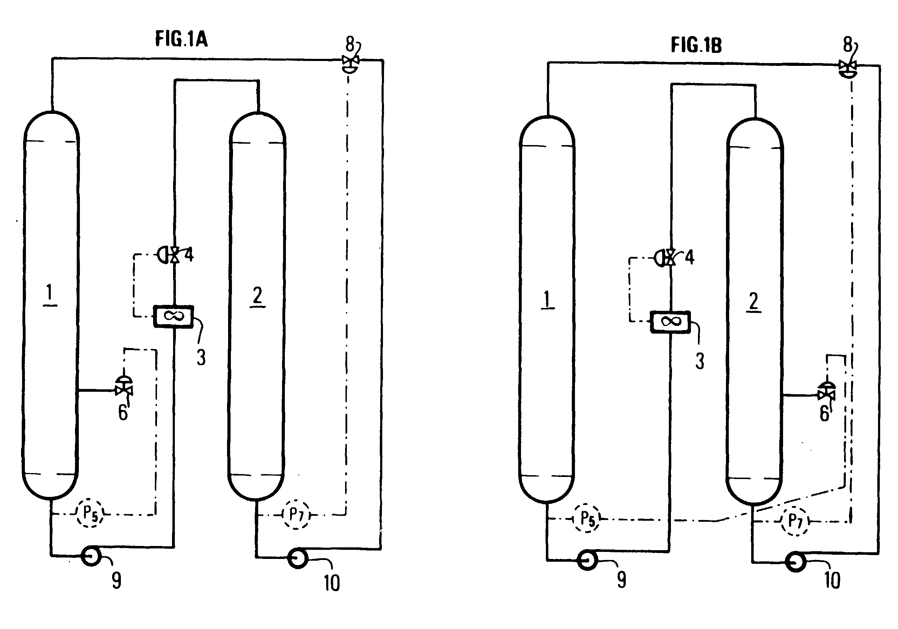

L'examen de ces figures permet de mieux comprendre la nature de ce problème. Les figures 1A et 1 B montrent comment sont régulées les pressions et les débits à l'intérieur de la boucle fermée des 24 lits constituant le lit mobile simulé. Deux adsorbeurs 1 et 2 comprennent chacun 12 lits de 1,3m de hauteur et 7,6m de diamètre. Deux pompes 9 et 10 permettent d'établir une circulation de liquide à l'intérieur des adsorbeurs. Un débitmètre 3 et une vanne de régulation de débit 4 permettent de contrôler avec une très bonne précision (0,2%) un débit compris entre 1100 et 3200 m3/h entre les adsorbeurs 1 et 2. Une vanne de prélèvement de raffinat 6 sous contrôle de pression permet de maintenir une pression de consigne 5 à l'aspiration de la pompe 9. Une vanne de contrôle 8 permet de maintenir une pression de consigne 7 à l'aspiration de la pompe 10. Par référence à un cycle de 24 périodes où le désorbant est injecté sur le premier lit de l'adsorbeur 1, la vanne de contrôle 6 se trouve en communication directe avec l'adsorbeur 2 au cours des périodes 1 à 3 et 16 à 24 (figure 1 b) et avec l'adsorbeur 1 au cours des périodes 4 à 15. Entre les périodes 3 et 4 le prélèvement de raffinat passe du fond de l'adsorbeur 2 à la tête de l'adsorbeur 1, entre les périodes 15 et 16 le prélèvement de raffinat passe du fond de l'adsorbeur 1 à la tête de l'adsorbeur 2. Des vannes tout ou rien permettant la mise en communication d'un lit déterminé avec le circuit de prélèvement mettent environ 2,5 secondes pour passer de la position ouverte à la position fermée (ou l'inverse). La perte de charge moyenne dans chacun des deux adsorbeurs est 4,2 bar. Lorsque la vanne de contrôle 6 est connectée au fond de l'un des deux adsorbeurs, son ouverture est environ 67%, lorsqu'elle est connectée en tête de l'un de l'un des adsorbeurs, son ouverture est environ 55%, le temps de manoeuvre pour passer de 67% d'ouverture à 55% d'ouverture est environ de 2,5 secondes. Selon l'art antérieur les meilleurs résultats sont obtenus en imposant à la vanne de contrôle 6 de passer à 55% d'ouverture environ 2 secondes avant la manoeuvre des vannes tout ou rien et en maintenant cette ouverture pendant environ 6 secondes, à l'issue de ces 6 secondes la vanne est reprise en automatique par le contrôleur. On note également que les meilleurs résultats sont obtenus avec des temps d'anticipation et de maintien légèrement différents au cours des deux transitions de fond à tête d'adsorbeur. Les vannes de contrôle 4 et 8 sont manoeuvrées selon le même principe avec toutefois des temps d'anticipation ou de retard et de maintien différents.

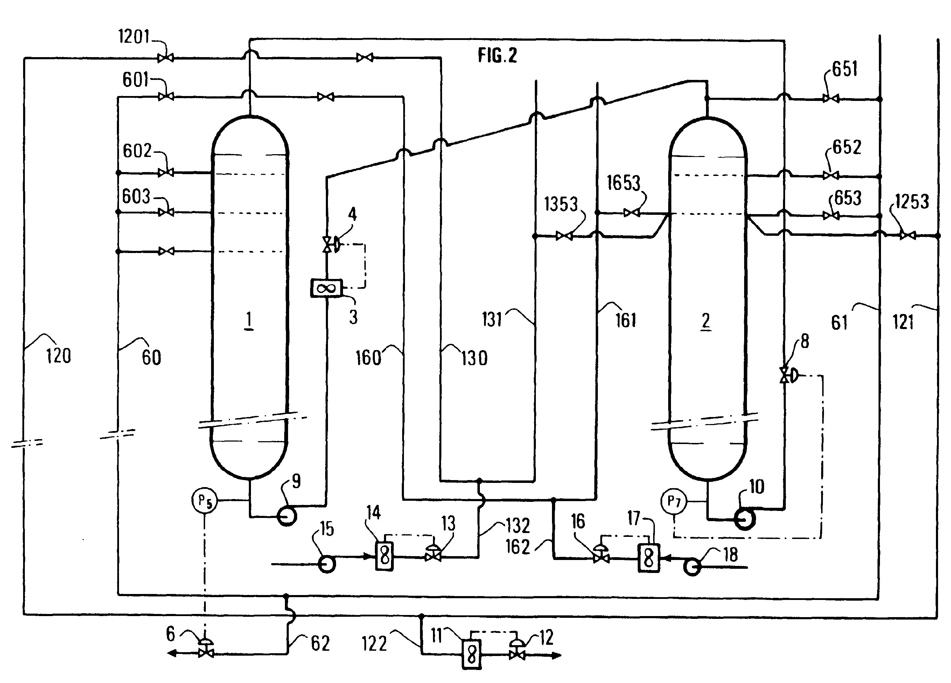

La figure 2 montre l'agencement des soutirages et des injections dans la boucle. Le circuit de prélèvement de raffinat comprend 24 vannes tout ou rien numérotées 601 à 612 et 651 à 662 (seules les vannes 601 à 603 et 651 à 653 sont représentées), ces 24 vannes sont reliées à la vanne de contrôle 6 de pression par l'intermédiaire des lignes 60, 61 et 62. Le circuit de prélèvement d'extrait comprend 24 vannes tout ou rien numérotées 1201 à 1212 et 1251 à 1262 (seules les vannes 1201 et 1253 sont représentées), ces 24 vannes sont reliées au débitmètre 11 et à la vanne de contrôle de débit 12 par l'intermédiaire des lignes 120, 121 et 122. Le circuit d'injection de charge comprend 24 vannes tout ou rien numérotées 1301 à 1312 et 1351 à 1362 (seule la vanne 1353 est représentée), ces 24 vannes sont reliées à une pompe 15, à un débitmètre 14 et à une vanne de contrôle de débit 13 par l'intermédiaire des lignes 130, 131 et 132. Le circuit d'injection de desorbant comprend 24 vannes tout ou rien numérotées 1601 à 1612 et 1651 à 1662 (seule la vanne 1653 est représentée), ces 24 vannes sont reliées à une pompe 18, à un débitmètre 17 et à une vanne de contrôle de débit 16 par l'intermédiaire des lignes 130,131 et 132.

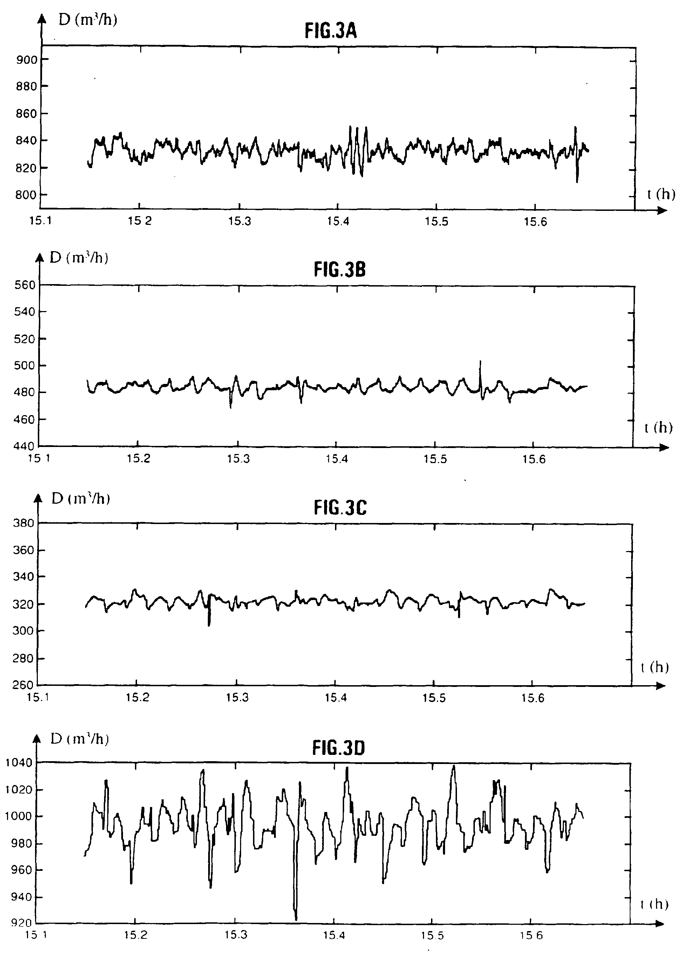

Les figures 3A, 3B, 3C et 3D présentent des enregistrements des quatre débits principaux pendant un cycle complet. Les consignes de débit de désorbant (fig. 3A), d'extrait (fig. 3C) et de charge (fig. 3B) sont respectivement de 830 m3/h, 320 m3/h et 480 m3/h. On constate que les amplitudes de variation autour de la consigne sont de plus ou moins 5m³/h pour l'extrait et la charge et de plus ou moins 5m3/h pour le désorbant. Le débit moyen résultant de raffinat (fig. 3D) est de 990 m3/h. Pour ce flux particulier les amplitudes de variation sont de plus ou moins 50 m3/h au lieu des 20 m3/h auxquels on pourrait s'attendre (somme des amplitudes de variation des autres débits). Dans ces conditions, on obtient une production de paraxylene de 74 t/h avec une pureté de 99,87% et un rendement de 94,5%

Ces perturbations sont de deux types:
1) à chaque fois que l'on permute le prélèvement de raffinat de l'amont à l'aval des pompes de recirculation, malgré le prépositionnement de la vanne de contrôle de raffinat et une anticipation de sa manoeuvre de manière à tenir compte et du temps de manoeuvre de quelques secondes et de la différence de pression de quelques bar, on observe que la pression régulée en fond de l'un des deux adsorbeurs et le débit de raffinat subissent une perturbation.
2) à chaque fois que l'on permute un autre des flux principaux de tête à fond d'adsorbeur, on observe une perturbation résiduelle minime sur le débit du flux en train de permuter, et une perturbation induite sur le débit de raffinat.

Les inconvénients de ces perturbations du débit de raffinat sont:
1) la colonne à distiller située en aval de la vanne de contrôle de raffinat admettant un débit puisé, il en résulte donc un surdimensionnement par rapport à un débit d'entrée beaucoup plus régulier.
2) un débit de raffinat puisé provoque des pulsations des débits internes particulièrement dans les zones 3 et 4 du lit mobile simulé. De manière à garantir que les constituants du raffinat ne puissent pas traverser la zone 4, on est obligé de baisser le débit en zone 4, c'est à dire d'augmenter le débit de désorbant par rapport à un fonctionnement idéal. De manière à ne pas baisser le débit en zone 3 (donc le débit de charge et en fin de compte la productivité de l'unité) on est obligé de tolérer une perte en constituant recherché dans l'extrait un peu plus forte que ce qui serait obtenu avec un fonctionnement idéal.

Il est donc clair que l'application de l'art antérieur ne permet pas d'obtenir un débit de raffinat très régulier, et que pour les autres flux, chaque changement d'étape donne lieu à une petite perturbation nettement observable sur les enregistrements des figures 3A, 3B, 3C, 3D.

L'objet de l'invention est de réduire au minimum ces perturbations résiduelles de débit au moyen d'au moins un dispositif mécanique simple. Plus généralement l'objet de l'invention est d'obtenir dans des unités de séparation en lit mobile simulé des débits d'appoint et de soutirage ainsi que des débits internes les plus constants possibles, et indirectement d'utiliser moins de désorbant et d'augmenter le rendement.

Un objet particulier de l'invention est d'assurer une pression sensiblement constante juste en amont des vannes de régulation des flux de soutirage du lit mobile simulé. Un second objet particulier de l'invention est d'assurer une pression sensiblement constante juste en aval des vannes de régulation des flux d'injection dans le lit mobile simulé.

Il est bien connu qu'une chaîne de régulation de débit ou de pression constituée d'un capteur, d'un régulateur généralement fonctionnant suivant l'algorithme Proportionnel Intégral Dérivé (PID) et d'une vanne doit assurer deux fonctions:
1) maintenir la mesure le plus près possible de la consigne tant que la dite consigne reste fixe (soit une rejection rapide des perturbations),
2) ramener la mesure à la valeur de la consigne le plus vite possible lorsque ladite consigne est changée (soit une poursuite rapide).

Dans le cas d'un lit mobile simulé puisque l'on soutire et que l'on injecte en séquence à partir de ou vers une série de lits, où un fluide s'écoule, c'est la pression amont des vannes de soutirage ou la pression aval des vannes d'injection qui varie en fonction de la position de l'injection ou du soutirage. La valeur de la consigne reste donc constante mais les conditions à l'une des bornes de la vanne de régulation variant, Le régulateur P.I.D. doit donc réajuster la position de la vanne à chaque permutation.

L'invention consiste donc à placer un ou plusieurs dispositifs mécaniques permettant d'abaisser la pression, judicieusement disposés sur le circuit de prélèvement sous contrôle de pression (généralement le raffinat) et de préférence à placer un ou plusieurs dispositifs mécaniques permettant d'abaisser la pression sur chacun des circuits de prélèvement et d'introduction dans la boucle de lits constituant le lit mobile simulé.

De manière plus précise, l'invention concerne une unité de séparation en lit mobile simulé d'au moins un isomère de xylène à partir d'une charge d'hydrocarbures en contenant comprenant une pluralité de lits disposés en boucle fermée dans au moins une colonne chromatographique, au moins un organe de recirculation de fluide, et comportant au moins deux lignes d'injection de fluide sous contrôle de débit et au moins deux lignes de soutirage de fluide, une des dites lignes de soutirage (extrait ou raffinat) étant sous contrôle de débit, l'autre étant sous contrôle de pression par l'intermédiaire d'au moins une vanne de contrôle,
caractérisée en ce que ladite ligne de soutirage sous contrôle de pression comporte au moins un organe de restriction, et de préférence plusieurs organes de restriction en parallèle ou en série, permettant d'abaisser la pression entre un au moins des lits et la vanne de contrôle de pression, ce ou ces organes étant conçus pour assurer, en amont de ladite vanne, une pression sensiblement constante ou bien située à l'intérieur d'une plage de valeurs comprises entre la valeur de pression de soutirage la plus faible PO et la valeur de PO augmentée d'une fraction de la perte de charge de la colonne.

Dans le cas d'unités pilotes ou de petites unités industrielles, un seul dispositif par flux prélevé ou injecté est nécessaire: il s'agit d'un régulateur de pression aval ou déverseur dans le cas d'un soutirage de fluide; pour une injection de fluide on utilisera un régulateur de pression amont. Ces dispositifs purement mécaniques biens connus de l'art antérieur sont essentiellement constitués d'un ressort et d'une membrane.

Dans le cas de grosses unités industrielles ce type de dispositif ne convient pas très bien: il est d'une part trop onéreux et d'autre part, son temps de réaction est important. Il est donc beaucoup plus pratique que la ligne de soutirage sous contrôle de pression comprenne plusieurs organes de restriction en parallèle ou en série de manière à maintenir la pression en amont de la vanne de contrôle des flux prélevés à l'intérieur d'une plage prédéterminée. De préférence cette ligne de soutirage peut comprendre une pluralité d'orifices calibrés pour assurer une pression sensiblement constante en aval d'une vanne de régulation d'un flux d'injection ou en amont d'une vanne de régulation d'un flux de soutirage. Selon une variante, la ligne de soutirage qui comporte une vanne de contrôle de pression comprend en tant qu'organe de restriction permettant d'abaisser la pression, au moins une vanne de régulation de la pression différentielle aux bornes de la vanne de contrôle de pression, la dite vanne de régulation de pression différentielle étant située en aval de la vanne de contrôle de pression et en dérivation de la ligne de soutirage.

Selon un mode préféré, la ligne de soutirage sous contrôle de pression comportant au moins un organe de restriction peut être la ligne de soutirage d'un raffinat.

L'autre ligne de soutirage peut comporter une vanne de contrôle de débit et au moins un organe de restriction situé entre au moins un des lits et l'entrée (amont) de la vanne de soutirage sous contrôle de débit. Comme variante, elle peut comporter une vanne de contrôle de débit et au moins une vanne de régulation de la pression différentielle aux bornes de la vanne de contrôle de débit, ladite vanne de régulation de pression différentielle étant située en aval de la vanne de contrôle de débit et en dérivation de la ligne de soutirage. Selon une autre caractéristique de l'invention, au moins une des lignes d'injection de fluide peut comporter une vanne de contrôle de débit et au moins un organe de restriction situé entre la sortie (aval) de la vanne de contrôle de débit et au moins un lit de manière à maintenir la pression en aval de la vanne de contrôle de débit à l'intérieur d'une plage de pressions prédéterminée.

Selon une variante, au moins une des lignes d'injection de fluide peut comporter une vanne de contrôle de débit et au moins une vanne de régulation de la pression différentielle aux bornes de la vanne de contrôle de débit, la dite vanne de régulation de pression différentielle étant située en amont de la vanne de contrôle de débit et en dérivation de la ligne d'injection. Cette vanne de régulation de pression différentielle peut ainsi constituer l'organe de restriction mentionné plus haut, dans ce cas particulier il est localisé en amont de la vanne de contrôle de débit au lieu d'être situé en aval.

L'invention concerne aussi un procédé de régulation de débits mettant en oeuvre le dispositif décrit selon la revendication 10, caractérisé en ce que l'on établit une perte de charge appropriée en disposant au moins un organe de restriction et de préférence plusieurs organes de restriction en parallèle ou en série, de taille adéquate, sur une ligne de soutirage d'un fluide de la ou des colonnes entre au moins un des lits et une vanne de contrôle de pression disposée sur ladite ligne de soutirage (extrait ou raffinat) de façon à obtenir une pression en amont de ladite vanne dans une plage de valeurs comprises entre la valeur de pression de soutirage la plus faible PO et la dite valeur PO augmentée d'une fraction de la perte de charge de la colonne, et de préférence égale à au plus la dite valeur PO augmentée de 50% de la perte de charge de la colonne et de manière particulièrement préférée égale à au plus la dite valeur PO augmentée de 10% de la perte de charge de la colonne.

Selon une caractéristique du procédé, on peut établir une perte de charge appropriée en disposant au moins un organe de restriction et de préférence plusieurs organes de restriction en parallèle ou en série, de taille adéquate, sur au moins une des lignes d'injection d'un fluide dans la ou les colonnes entre la vanne de contrôle de débit disposée sur ladite ligne d'injection (charge ou désorbant) et entre au moins un des lits de façon à obtenir une pression en aval de ladite vanne dans une plage de valeurs comprises entre la valeur de pression d'injection la plus forte PM et la dite valeur PM diminuée d'une fraction de la perte de charge de la colonne, et de préférence égale à au moins la dite valeur PM diminuée de 50% de la perte de charge de la colonne et de manière particulièrement préférée égale à au moins la dite valeur PM diminuée de 10% de la perte de charge de la colonne.

Selon une variante, on peut réguler la perte de charge aux bornes d'au moins une vanne de contrôle de débit disposée sur au moins une des lignes d'injections (charge ou désorbant) en plaçant une vanne de régulation de pression différentielle en amont de la vanne de contrôle de débit, en dérivation de la ligne d'injection de fluide, de façon à obtenir une différence de pression aux bornes de la vanne de régulation de débit comprise entre une valeur de référence et la dite valeur de référence augmentée d'une fraction de la perte de charge de la colonne, et de préférence égale à au plus la dite valeur de référence augmentée de 50% de la perte de charge de la colonne et de manière particulièrement préférée égale à au plus à la dite valeur de référence augmentée de 10% de la perte de charge de la colonne, la dite valeur de référence étant habituellement comprise entre 0,2 et 10 bar et plus particulièrement entre 0,5 et 2 bar.

Selon une autre caractéristique du procédé, on peut établir une perte de charge appropriée en disposant au moins un organe de restriction et de préférence plusieurs organes de restriction en parallèle ou en série, de taille adéquate, sur une ligne de soutirage d'un fluide de la ou des colonnes entre au moins un des lits et une vanne de contrôle de débit disposée sur ladite ligne de soutirage (extrait ou raffinat) de façon à obtenir une pression en amont de ladite vanne dans une plage de valeurs comprises entre la valeur de pression de soutirage la plus faible P0 et la dite valeur P0 augmentée d'une fraction de la perte de charge de la colonne, et de préférence égale à au plus la dite valeur P0 augmentée de 50% de la perte de charge de la colonne et de manière particulièrement préférée égale à au plus la dite valeur P0 augmentée de 10% de la perte de charge de la colonne.

La perte de charge crée par un orifice de restriction est proportionnelle à K r v², où K est une constante, r est la masse volumique du fluide et v la vitesse linéaire dans l'orifice. Généralement, on peut considérer que la vitesse maximale tolérable dans l'orifice est de l'ordre de 15 mètres par seconde, et que la perte de charge maximale crée par un orifice calibré n'excède pas 1 bar environ.

### On peut mettre en oeuvre le procédé de la façon suivante:

La manière la plus simple de procéder serait de mettre à chaque lit, entre chaque vanne tout ou rien et la ligne commune de prélèvement ou de soutirage un nombre d'orifices de restriction dépendant de chacun des niveaux de pression et de soutirage. Par exemple si la perte de charge par lit est de 0,35 bar et si douze lits sont disposés dans chacune des colonnes, pour obtenir une pression constante en amont d'une vanne de contrôle de soutirage, pour le soutirage du premier des lits on dispose en série cinq orifices assurant chacun une perte de charge de 0,7 bar et un orifice assurant une perte de charge de 0,35 bar. Pour le soutirage du second des lits on dispose en série cinq orifices assurant chacun une perte de charge de 0,7 bar. Pour le soutirage du troisième des lits on dispose en série quatre orifices assurant chacun une perte de charge de 0,7 bar et un orifice assurant une perte de charge de 0,35 bar. Pour le soutirage du quatrième des lits on dispose en série quatre orifices assurant chacun une perte de charge de 0,7 bar. Pour le soutirage du cinquième des lits on dispose en série trois orifices assurant chacun une perte de charge de 0,7 bar et un orifice assurant une perte de charge de 0,35 bar. Pour le soutirage du sixième des lits on dispose en série trois orifices assurant chacun une perte de charge de 0,7 bar. Pour le soutirage du septième des lits on dispose en série deux orifices assurant chacun une perte de charge de 0,7 bar et un orifice assurant une perte de charge de 0,35 bar. Pour le soutirage du huitième des lits on dispose en série deux orifices assurant chacun une perte de charge de 0,7 bar. Pour le soutirage du neuvième des lits on dispose en série un orifice assurant chacun une perte de charge de 0,7 bar et un orifice assurant une perte de charge de 0,35 bar. Pour le soutirage du dixième des lits on dispose un orifice assurant une perte de charge de 0,7 bar. Pour le soutirage du onzième des lits on dispose un orifice assurant une perte de charge de 0,35 bar. Soit 36 orifices calibrés par colonne et par courant injecté ou soutiré. Le nombre total d'orifices de restriction pour la totalité de la boucle serait donc de 288.
Fort heureusement, dans le cas précédemment cité, une disposition plus judicieuse permet de réduire de 36 à 11 le nombre d'orifices de restriction par colonne et par courant injecté ou soutiré: pour le soutirage du premier des lits on dispose un orifice assurant chacun une perte de charge de 0,35 bar. Pour le soutirage du second des lits on ne dispose aucun orifice, sur la ligne commune entre le second et le troisième lit on dispose un orifice assurant une perte de charge de 0,7 bar. Pour le soutirage du troisième des lits on dispose un orifice assurant une perte de charge de 0,35 bar. Pour le soutirage du quatrième des lits on ne dispose aucun orifice, sur la ligne commune entre le quatrième et le cinquième lit on dispose un orifice assurant une perte de charge de 0,7 bar. Pour le soutirage du cinquième des lits on dispose un orifice assurant une perte de charge de 0,35 bar, pour le soutirage du sixième des lits on ne dispose aucun orifice, sur la ligne commune entre le sixième et le septième lit on dispose un orifice assurant une perte de charge de 0,7 bar. Pour le soutirage du septième des lits on dispose un orifice assurant chacun une perte de charge de 0,35 bar.
Pour le soutirage du huitième des lits on ne dispose aucun orifice, sur la ligne commune entre le huitième et le neuvième lit on dispose un orifice assurant une perte de charge de 0,7 bar. Pour le soutirage du neuvième des lits on dispose un orifice assurant une perte de charge de 0,35 bar. Pour le soutirage du dixième des lits on ne dispose aucun orifice, sur la ligne commune entre le dixième et le onzième lit on dispose un orifice assurant une perte de charge de 0,7 bar. Pour le soutirage du onzième des lits on dispose un orifice assurant une perte de charge de 0,35 bar.

Selon l'étendue tolérée de variation de pression, le nombre de lits, le nombre de pompes de recirculation dans la boucle, et le temps de manoeuvre de la vanne de contrôle, on pourra disposer de 2 à 30 orifices calibrés par circuit de prélèvement.

L'invention sera mieux comprise au vu des exemples suivants:

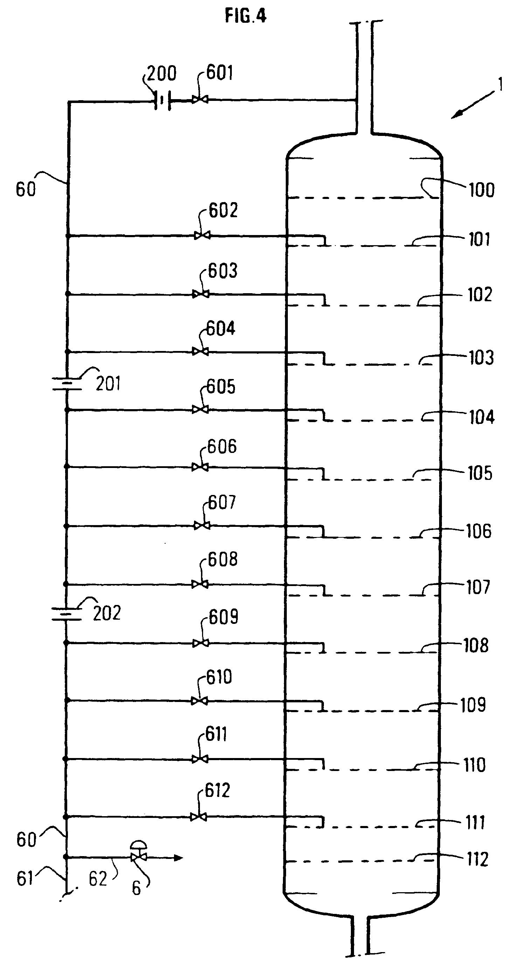

Exemple 1: La figure 4 présente un exemple de réalisation selon l'invention dans le cas où l'on tolère environ 1 bar de variation de pression en amont de la vanne de contrôle de raffinat: il suffit de 6 orifices calibrés pour obtenir une pression comprise entre 8,35 et 9,4 bar. Seul l'adsorbeur 1 est représenté sur la figure 4.
La perte de charge pour un débit interne moyen d'environ 2000 m3/h est de 4,2 bar, la perte de charge moyenne à travers chacun des lits est d'environ 0,35 bar, cette perte de charge se répartit à raison d'environ 0,27 bar pour le tamis moléculaire et 0,08 bar pour traverser l'un des plateaux 101 à 111. Les plateaux 101 à 111 contiennent en outre une série de trous de distribution par flux principal injecté ou soutiré. Le diamètre de ces trous et celui des lignes mettant en communication les trous à chacune des quatre brides de raccordement vers l'extérieur de l'adsorbeur ont été calculés pour que la perte de charge entre l'intérieur et l'extérieur de l'adsorbeur soit de 1 bar. La vanne 601 de soutirage constitue une singularité puisqu'elle est directement en communication avec le refoulement de la pompe de recirculation 10. Selon l'invention on a placé trois orifices calibrés 200, 201 et 202 sur la ligne 60. (les orifices correspondants sont placés sur la ligne 61 reliant l'adsorbeur 2 (figure 2) à la vanne de contrôle 6 ne sont pas représentés sur la figure) L'orifice calibré 200 réalise une perte de charge de 1 bar, tandis que les orifices calibrés 201 et 202 réalisent chacun une perte de charge de 1,4 bar. Pour simplifier la description on a appelé " l'orifice calibré 201 et l'orifice calibré 202 " une série de deux orifices calibrés réalisant chacun une perte de charge de 0,7 bar . Le bilan pression suivant la position du prélèvement de raffinat est donné ci dessous:
période 4: le raffinat est prélevé par la vanne 601: pression dans la ligne de recirculation 13,2 bar, pression en aval de l'orifice 200 12,2 bar, pression en aval de l'orifice 201 10,8 bar, pression en aval de l'orifice 202 9,4 bar.
période 5: le raffinat est prélevé par la vanne 602: pression dans l'adsorbeur au niveau du plateau 101 12,85 bar, pression en amont de l'orifice 201 11,85 bar, pression en aval de l'orifice 201 10,45 bar, pression en aval de l'orifice 202 9,05 bar.
période 6: le raffinat est prélevé par la vanne 603: pression dans l'adsorbeur au niveau du plateau 102 12,5 bar, pression en amont de l'orifice 201 11,5 bar, pression en aval de l'orifice 201 10,1 bar, pression en aval de l'orifice 202 8,65 bar.
période 7: le raffinat est prélevé par la vanne 604: pression dans l'adsorbeur au niveau du plateau 103 12,15 bar, pression en amont de l'orifice 201 11,15 bar, pression en aval de l'orifice 201 9,75 bar, pression en aval de l'orifice 202 8,35 bar.
période 8: le raffinat est prélevé par la vanne 605: pression dans l'adsorbeur au niveau du plateau 104 11,8 bar, pression en amont de l'orifice 202 10,8 bar, pression en aval de l'orifice 202 9,4 bar.
période 9: le raffinat est prélevé par la vanne 606: pression dans l'adsorbeur au niveau du plateau 105 11,45 bar, pression en amont de l'orifice 202 10,45 bar, pression en aval de l'orifice 202 9,05 bar.
période 10: le raffinat est prélevé par la vanne 607: pression dans l'adsorbeur au niveau du plateau 106 11,1 bar, pression en amont de l'orifice 202 10,1 bar, pression en aval de l'orifice 202 8,7 bar.
période 11: le raffinat est prélevé par la vanne 608: pression dans l'adsorbeur au niveau du plateau 107 10,75 bar, pression en amont de l'orifice 202 9,75 bar, pression en aval de l'orifice 202 8,35 bar.
période 12: le raffinat est prélevé par la vanne 609: pression dans l'adsorbeur au niveau du plateau 108 10,4 bar, pression en aval de l'orifice 202 9,4 bar.
période 13: le raffinat est prélevé par la vanne 610: pression dans l'adsorbeur au niveau du plateau 109 10,05 bar, pression en aval de l'orifice 202 9,05 bar.
période 14: le raffinat est prélevé par la vanne 611: pression dans l'adsorbeur au niveau du plateau 110 9,7 bar, pression en aval de l'orifice 202 8,7 bar.
période 15: le raffinat est prélevé par la vanne 612: pression dans l'adsorbeur au niveau du plateau 111 9,35 bar, pression en aval de l'orifice 202 8,35 bar. La pression mesurée entre l'orifice 202 et la vanne de contrôle 6 varie entre 8,35 et 9,4 bar. L'ouverture de la vanne de contrôle 64,5 à 67%. Le prépositionnement avec maintien de la vanne pendant 5 secondes intervient entre les étapes 3 et 4, 7 et 8, 11 et 12, 15 et 16, 19 et 20, 23 et 24. La fluctuation de débit de raffinat observée autour de la valeur moyenne est environ +/- 30 m3/h. Avec cette modification, on réduit les débits de désorbant et de raffinat de 20 m3/h tout en gardant des performances identiques, on constate également que la perte en désorbant en tête de la colonne à distiller de raffinat se trouve également réduite d'environ 30%, grâce au fonctionnement plus stable.

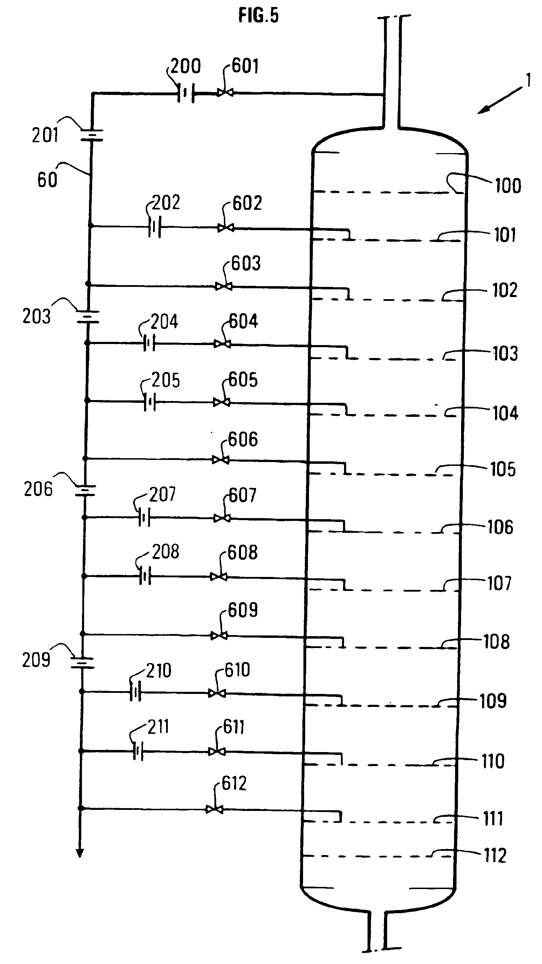

Exemple 2: La figure 5 présente un exemple de réalisation selon l'invention dans le cas où l'on tolère environ 0,1 bar de variation de pression juste en amont de la vanne de contrôle de raffinat 6: il faut alors 24 orifices calibrés pour obtenir une pression de l'ordre de 8,35 bar en amont de la dite vanne. Selon l'invention on a placé douze orifices calibrés 200 à 211 sur la ligne 60. (les orifices symétriques correspondants placés sur la ligne 61 reliant l'adsorbeur 2 à la vanne de contrôle 6 ne sont pas représentés sur la figure). Les orifices calibrés 203,206 et 209 réalisent chacun une perte de charge de 1,05 bar, l'orifice calibré 201 réalise une perte de charge de 1 bar, les orifices calibrés 200,204,207 et 211 réalisent chacun une perte de charge de 0,7 bar, les orifices calibrés 202, 205,208 et 212 réalisent chacun une perte de charge de 0,35 bar. Le bilan pression suivant la position du prélèvement de raffinat est donné ci dessous: période 4: le raffinat est prélevé par la vanne 601: pression dans la ligne de recirculation 13,2 bar, pression en aval des l'orifices 200 et 201,11,5 bar, pression en aval de l'orifice 203 10,45 bar, pression en aval de l'orifice 206 9,4 bar, pression en aval de l'orifice 209 8,35 bar.
période 5: le raffinat est prélevé par la vanne 602: pression dans l'adsorbeur au niveau du plateau 101 12,85 bar, pression en amont de l'orifice 202 11,85 bar, pression en aval de l'orifice 202 11,5 bar, pression en aval de l'orifice 203 10,45 bar, pression en aval de l'orifice 206 9,4 bar, pression en aval de l'orifice 209 8,35 bar.
période 6: le raffinat est prélevé par la vanne 603: pression dans l'adsorbeur au niveau du plateau 102 12,5 bar, pression en amont de l'orifice 203 11,5 bar, pression en aval de l'orifice 203 10,45 bar, pression en aval de l'orifice 206 9,4 bar, pression en aval de l'orifice 209 8,35 bar.
période 7: le raffinat est prélevé par la vanne 604: pression dans l'adsorbeur au niveau du plateau 103 12,15 bar, pression en amont de l'orifice 204 11,15 bar, pression en aval de l'orifice 204 10,45 bar, pression en aval de l'orifice 206 9,4 bar, pression en aval de l'orifice 209 8,35 bar.
période 8: le raffinat est prélevé par la vanne 605: pression dans l'adsorbeur au niveau du plateau 104 11,8 bar, pression en amont de l'orifice 205 10,8 bar, pression en aval de l'orifice 205 10,45 bar, pression en aval de l'orifice 206 9,4 bar, pression en aval de l'orifice 209 8,35 bar.
période 9: le raffinat est prélevé par la vanne 606: pression dans l'adsorbeur au niveau du plateau 105 11,45 bar, pression en amont de l'orifice 206 10,45 bar, pression en aval de l'orifice 206 9,4 bar, pression en aval de l'orifice 209 8,35 bar.
période 10: le raffinat est prélevé par la vanne 607: pression dans l'adsorbeur au niveau du plateau 106 11,1 bar, pression en amont de l'orifice 207 10,1 bar, pression en aval de l'orifice 207 9,4 bar, pression en aval de l'orifice 209 8,35 bar. période 11: le raffinat est prélevé par la vanne 608: pression dans l'adsorbeur au niveau du plateau 107 10,75 bar, pression en amont de l'orifice 208 9,75 bar, pression en aval de l'orifice 208 9,4 bar, pression en aval de l'orifice 209 8,35 bar. période 12: le raffinat est prélevé par la vanne 609: pression dans l'adsorbeur au niveau du plateau 107 10,4 bar, pression en amont de l'orifice 209 9,4 bar, pression en aval de l'orifice 209 8,35 bar.
période 13: le raffinat est prélevé par la vanne 610: pression dans l'adsorbeur au niveau du plateau 108 10,05 bar, pression en amont de l'orifice 211 9,05 bar, pression en aval de l'orifice 211 8,35 bar.
période 14: le raffinat est prélevé par la vanne 611: pression dans l'adsorbeur au niveau du plateau 109 9,7 bar, pression en amont de l'orifice 212 8,7 bar, pression en aval de l'orifice 212 8,35 bar.
période 15: le raffinat est prélevé par la vanne 612: pression dans l'adsorbeur au niveau du plateau 110 9,35 bar, pression en amont de la vanne de régulation 6 8,35 bar.
La pression mesurée entre juste en amont de la vanne de contrôle 6 est de 8,35 bar avec des fluctuations inférieures à 0,1 bar. L'ouverture de la vanne de contrôle est environ 67%. Le prépositionnement de la vanne contrôle 6 n'est plus nécessaire. La fluctuation de débit de raffinat observée autour de la valeur moyenne est environ +/-25 m3/h. L'amélioration par rapport à l'exemple 1 n'est pas très importante: les perturbations résiduelles proviennent essentiellement de la somme des fluctuations des autres flux.

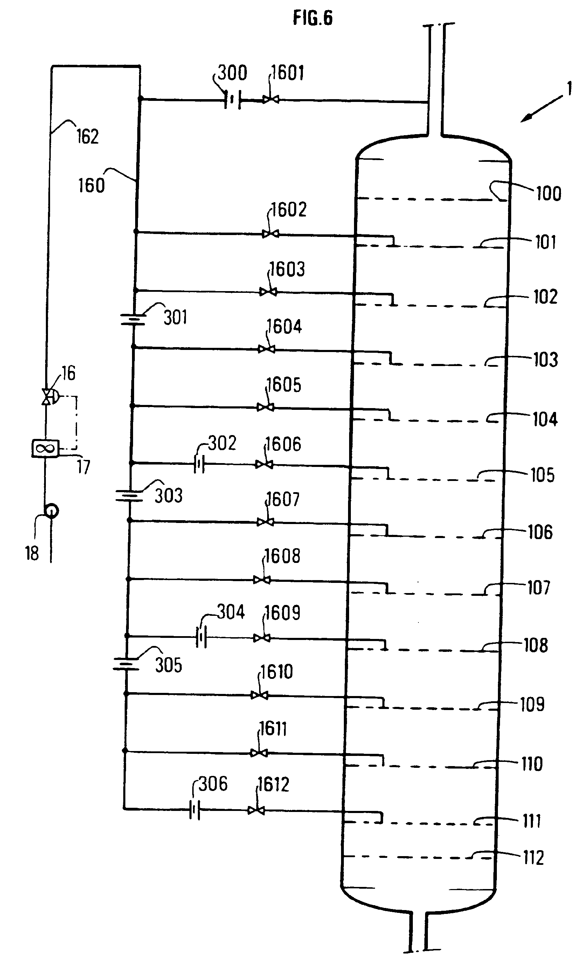

Exemple 3: Toutes les injections et tous les soutirages sont équipés selon l'invention: sur le circuit d'extrait on tolère une variation de pression de l'ordre de 1 bar, on place donc 6 orifices calibrés sur ce circuit, sur le circuit de raffinat on ne tolère qu'une variation de 0,35 bar on place donc 12 orifices calibrés sur ce circuit. Sur le circuit de charge on tolère une variation de pression de l'ordre de 1 bar, on place donc 4 orifices calibrés sur ce circuit: en effet l'équivalent de l'orifice 200 sur la figure 4 n'est pas nécessaire dans ce cas. La figure 6 présente un exemple de réalisation selon l'invention où l'on ne tolère qu'une variation de pression de 0,35 bar en aval de la vanne de contrôle de désorbant 16. Seul l'adsorbeur 1 est représenté avec ses 7 orifices notés 300 à 306 et le circuit d'injection de désorbant comporte 14 orifices. Les orifices calibrés 300, 302, 304, 306 réalisent chacun une perte de charge de 0,35 bar, l'orifice calibré 301 réalise une perte de charge de 0,7 bar, les orifices calibrés 303 et 305 réalisent chacun une perte de charge de 1,05 bar. Pour simplifier la description on a appelé "l'orifice calibré 303" une série de deux orifices calibrés réalisant chacun une perte de charge de 0,525 bar . On remarquera en outre que la ligne 162 est connectée à la ligne 160 en partie haute et non plus en partie basse comme dans la figure 2 . Le bilan pression suivant la position du prélèvement de raffinat est donné ci dessous:
période 1: le désorbant est injecté par la vanne 1601: pression dans la ligne de recirculation 13,2 bar, pression en amont de l'orifice 300 13,5 bar.
période 2: le désorbant est injecté par la vanne 1602 : pression dans l'adsorbeur au niveau du plateau 101 12,85 bar, pression au niveau de la vanne 1602 13,85 bar.
période 3: le désorbant est injecté par la vanne 1603: pression dans l'adsorbeur au niveau du plateau 102 12,5 bar, pression au niveau de la vanne 1603 13,5 bar.
période 4: le désorbant est injecté par la vanne 1604: pression dans l'adsorbeur au niveau du plateau 103 12,15 bar, pression au niveau de la vanne 1604 13,15 bar, pression en amont de l'orifice 301 13,85 bar.
période 5: le désorbant est injecté par la vanne 1605: pression dans l'adsorbeur au
niveau du plateau 104 11,8 bar, pression au niveau de la vanne 1605 12,8 bar, pression en amont de l'orifice 301 13,5 bar.
période 6: le désorbant est injecté par la vanne 1606: pression dans l'adsorbeur au niveau du plateau 105 11,45 bar, pression au niveau de la vanne 1606 12,45 bar, pression en amont de l'orifice 302 12,8 bar, pression en amont de l'orifice 301 13,5 bar.
période 7: le désorbant est injecté par la vanne 1607: pression dans l'adsorbeur au niveau du plateau 106 11,1 bar, pression au niveau de la vanne 1607 12,1 bar, pression en amont de l'orifice 303 13,15 bar, pression en amont de l'orifice 301 13,85 bar.
période 8: le désorbant est injecté par la vanne 1608: pression dans l'adsorbeur au niveau du plateau 107 10,75 bar, pression au niveau de la vanne 1608 11,75 bar, pression en amont de l'orifice 303 12,8 bar, pression en amont de l'orifice 301 13,5 bar.
période 9: le désorbant est injecté par la vanne 1609: pression dans l'adsorbeur au niveau du plateau 108 10,4 bar, pression au niveau de la vanne 1609 11,4 bar, pression en amont de l'orifice 304 11,75 bar, pression en amont de l'orifice 303 12,8 bar, pression en amont de l'orifice 301 13,5 bar.
période 10 : le désorbant est injecté par la vanne 1610 : pression dans l'adsorbeur au niveau du plateau 109 10,05 bar, pression au niveau de la vanne 1610 11,05 bar, pression en amont de l'orifice 305 12,1 bar, pression en amont de l'orifice 303 13,15 bar, pression en amont de l'orifice 301 13,85 bar.
période 11 : le désorbant est injecté par la vanne 1611 : pression dans l'adsorbeur au niveau du plateau 110 9,7 bar, pression au niveau de la vanne 1611 10,7 bar, pression en amont de l'orifice 305 11,75 bar, pression en amont de l'orifice 303 12,8 bar, pression en amont de l'orifice 301 13,5 bar. période 12 : le désorbant est injecté par la vanne 1612 : pression dans l'adsorbeur au niveau du plateau 111 9,35 bar, pression au niveau de la vanne 1612 10,35 bar, pression en amont de l'orifice 306 10,7 bar, pression en amont de l'orifice 305 11,75 bar, pression en amont de l'orifice 303 12,8 bar, pression en amont de l'orifice 301 13,5 bar.

La pression mesurée juste en aval de la vanne de contrôle 16 est comprise entre 13,5 et 13,85 bar. L'ouverture de la vanne de contrôle 16 est comprise entre 46 et 47%. Le prépositionnement de la vanne contrôle 16 n'est plus nécessaire. Le prépositionnement des vannes de contrôle 4, 8, 12 et 13 demeure nécessaire.

La fluctuation de débit de raffinat observée autour de la valeur moyenne est 20 environ +/- 10 m3/h. La fluctuation de débit de désorbant observée autour de la valeur moyenne est inférieure à +/- 5 m3/h. Les fluctuations des débits de charge et d'extrait observée autour de la valeur moyenne sont environ +/- 2 m3/h.

L'amélioration par rapport à l'art antérieur est très nette: en baissant les débits de désorbant et de raffinat de 40 m3/h, tout en maintenant constants les débits internes dans les zones 1, 2 et 3 on garde une pureté identique et l'on observe une augmentation de rendement de 0,45%, il en résulte que la production augmente de 0,3 t/h.

L'invention comporte un autre avantage: lors de la permutation d'un lit au suivant, pendant un court instant (environ 2 secondes) il y a la possibilité de by-pass entre ces deux lits (on attend que la vanne suivante soit ouverte avant de refermer la précédente). Pour éviter ce by-pass non souhaité on peut installer un clapet anti-retour entre chaque vanne tout ou rien et les lignes communes d'injection et de soutirage, ce qui poserait les problèmes suivants: Ces clapets sont mécaniquement très sollicités (grand nombre de cycles entre deux arrêts) et peuvent être endommagés par les à-coup qu'ils subissent, en particulier ceux disposés sur le lit où la pression est la plus faible pour les soutirages et ceux disposés sur le lit où la pression est la plus forte pour les injections subissent des chocs très violents comparés à tous les autres. Des orifices de restriction permettent sinon de supprimer totalement la possibilité de by-pass, tout au moins de la limiter considérablement.

Lorsque l'unité est poussée à son maximum de capacité (typiquement 110 à 120%) de sa capacité nominale ou lorsqu'au contraire elle doit tourner à son minimum de capacité (typiquement 55 à 60% de sa capacité nominale), le ratio des débits dans ces deux cas varie du simple au double. En première approximation, la perte de charge dans les lits est proportionnelle aux débits. Il en résulte que les orifices de restriction adaptés à la capacité nominale ne remplissent plus totalement leur rôle. Lorsqu'un fonctionnement de longue durée à une capacité significativement différente de la capacité nominale est prévu, il est intéressant de les remplacer par un autre jeu d'orifices adaptés aux nouveaux débits opératoires.

L'application de l'invention a été décrite ci-dessus pour les unités comprenant une pluralité de vannes tout ou rien réalisant les injections et soutirages. L'invention peut également s'appliquer aux unités de type "Sorbex". Sur ces unités une vanne dite "rotative " met en communication d'une part chacun des lits du lit mobile simulé et d'autre part de 4 à 7 flux d'injection et de soutirage. Une des particularités des unités de type sorbex est de ne comporter qu'une seule ligne de liaison par lit. Dans le cas d'une unité à 24 lits disposés en deux adsorbeurs de 12 lits par exemple, il faudrait au moins 16 orifices disposés sur les 16 sorties dont le niveau de pression est le plus élevé pour obtenir une variation de pression de l'ordre de 1 bar en amont des vannes de contrôle de raffinat et d'extrait. Pour les circuits d'injection la différence de pression en aval des vannes de contrôle de charge et de désorbant serait alors nettement augmentée, puisque pour injecter dans les lits en tête d'adsorbeur, on trouverait les pressions les plus élevées de la boucle, qui seraient encore augmentées des pertes de charges des orifices de restriction, il en résulterait bien sûr des inconvénients évidents pour l'homme de l'art.

Deux alternatives se présentent donc: grâce à une modification de ce type d'unité on peut utiliser le dispositif préféré de l'invention; une autre possibilité consiste à utiliser une variante de l'invention.

Pour utiliser le dispositif préféré de l'invention, il faut utiliser deux vannes rotatives:
l'une dédiée aux injections, et l'autre aux soutirages. De plus deux lignes par lit sont nécessaires: l'une reliant le lit à la vanne rotative des injections, et l'autre à la vanne rotative des soutirages. De cette manière on peut placer les orifices de restriction adaptés au soutirage sur les lignes reliant chacun des lits à la vanne rotative dédiée
aux soutirages, et, indépendamment on peut placer les orifices de restriction adaptés aux injections sur les lignes reliant chacun des lits à la vanne rotative dédiée à l'injection.

Une variante de l'invention permet d'utiliser en tant qu'organe de restriction une vanne de régulation de la pression différentielle aux bornes d'au moins une et de préférence aux bornes de chacune des vannes de contrôle de débit ou de pression. Naturellement, quel que soit le type d'unité en lit mobile simulé, les solutions consistant à panacher le dispositif préféré de l'invention et la variante sont toujours possibles.

On remarquera qu'à débit constant et ouverture constante d'une vanne de régulation (par exemple quand elle est en mode manuel), si une perturbation de pression se produit en aval de la vanne elle se trouve répercutée en amont (et inversement). Pour une vanne d'injection de liquide qui doit travailler à débit constant et avec un pourcentage d'ouverture le plus constant possible, une régulation de la pression différentielle aux bornes de la vanne de contrôle de débit permet d'éviter de placer une série d'orifices de restriction sur les lignes d'injection par exemple (dans cas des unités de type Sorbex sur chacune des lignes reliant chaque lit à la vanne rotative). Dans ce cas, les différents niveaux de pression seront retransmis en amont des vannes de contrôle de débit des injections.

Aux bornes de la chaîne de régulation de débit de désorbant constituée du débitmètre et de la vanne de contrôle, on installe un capteur de pression différentielle. Cette mesure attaque une seconde vanne de contrôle qui permet de renvoyer du liquide en amont de la pompe de désorbant. Lorsque la vanne rotative avance d'une position, la baisse ou l'augmentation de pression entre deux lits consécutifs sera répercutée en amont de la vanne de contrôle de débit. Une ouverture ou une fermeture de la vanne de contrôle de pression différentielle permettra donc de maintenir la perte de charge constante à travers la vanne de contrôle de débit. Dans le cas particulier où l'injection de désorbant passe de l'amont à l'aval d'une des pompes de recyclage, le prépositionnement de vanne décrit dans l'art antérieur sera bien sûr utilisé. L'adjonction de la vanne de contrôle de pression différentielle ne consiste pas juste à déplacer le problème d'une vanne vers une autre strictement identique: en effet comme on régule une pression de liquide par une dérivation depuis la ligne d'injection, un faible soutirage de liquide entre la pompe et la vanne de contrôle de débit suffit à faire baisser la pression en amont de la vanne de débit.

Les soutirages peuvent être traités d'une manière similaire: un contrôle de pression différentielle aux bornes de la vanne de contrôle sera réalisé en plaçant en aval de la dite vanne de contrôle, en dérivation, une seconde vanne de contrôle.

Cependant vis à vis du dispositif préféré de l'invention (une série d'orifices) qui réagit de manière instantanée, une chaîne de régulation de pression différentielle présente toujours un temps de réaction, et de petites oscillations résiduelles de débit persistent, de plus les actions de deux vannes de contrôles couplées sont plus délicates à régler que celles d'une seule vanne.

## Revendications

1. Unité de séparation en lit mobile simulé d'au moins un isomère de xylène à partir d'une charge d'hydrocarbures en contenant comprenant une pluralité de lits disposés en boucle fermée dans au moins une colonne (1) chromatographique, au moins un organe de recirculation (9) de fluide, et comportant au moins deux lignes d'injection de fluide sous contrôle de débit et au moins deux lignes de soutirage de fluide, une des dites lignes de soutirage (extrait ou raffinat) étant sous contrôle de débit, l'autre (60) étant sous contrôle de pression par l'intermédiaire d'au moins une vanne (6) de contrôle,
**caractérisée en ce que** ladite ligne (60) de soutirage sous contrôle de pression comporte au moins un organe de restriction (201), et de préférence plusieurs organes de restriction en parallèle ou en série, permettant d'abaisser la pression entre un au moins des lits et la vanne de contrôle de pression, ce ou ces organes étant conçus pour assurer, en amont de ladite vanne, une pression sensiblement constante ou bien située à l'intérieur d'une plage de valeurs comprises entre la valeur de pression de soutirage la plus faible PO et la valeur de PO augmentée d'une fraction de la perte de charge de la colonne.

2. Unité de séparation selon la revendication 1 dans laquelle ladite fraction de la perte de charge de la colonne est égale à 50% de la perte de charge de la colonne.

3. Unité de séparation selon la revendication 2 dans laquelle ladite fraction de la perte de charge de la colonne est égale à 10% de la perte de charge de la colonne.

4. Unité de séparation selon l'une des revendications 1 à 3 dans laquelle la ligne de soutirage sous contrôle de pression contient le raffinat.

5. Unité de séparation selon l'une des revendications 1 à 4 dans laquelle au moins une des lignes (130) d'injection comporte une vanne (13) de contrôle de débit et au moins un organe de restriction situé entre la sortie (aval) de la vanne de contrôle de débit et au moins un lit de manière à maintenir la pression en aval de la vanne de contrôle de débit sensiblement constante ou à l'intérieur d'une plage de valeurs comprises entre la valeur de pression d'injection la plus forte PM et la valeur de PM diminuée de 50% de la perte de charge de la colonne.

6. Unité de séparation selon l'une des revendications 1 à 4 dans laquelle au moins une des lignes d'injection comporte une vanne de contrôle de débit et au moins une vanne de régulation de la pression différentielle aux bornes de la vanne de contrôle de débit, la dite vanne de régulation de pression différentielle étant située en amont de la vanne de contrôle de débit et en dérivation de la ligne d'injection.

7. Unité de séparation selon l'une des revendications 1 à 6 dans laquelle l'autre ligne (120) de soutirage comporte une vanne (12) de contrôle de débit et au moins un organe de restriction situé entre au moins un des lits et l'entrée (amont) de la vanne de soutirage sous contrôle de débit.

8. Unité de séparation selon l'une des revendications 1 à 6, dans laquelle l'autre ligne de soutirage comporte une vanne de contrôle de débit et au moins une vanne de régulation de la pression différentielle aux bornes de la vanne de contrôle de débit, ladite vanne de régulation de pression différentielle étant située en aval de la vanne de contrôle de débit et en dérivation de la ligne de soutirage.

9. Unité de séparation selon l'une des revendications 1 à 8 dans laquelle ladite ligne de soutirage qui comporte une vanne de contrôle de pression comprend en tant qu'organe de restriction permettant d'abaisser la pression, au moins une vanne de régulation de la pression différentielle aux bornes de la vanne de contrôle de pression, la dite vanne de régulation de pression différentielle étant située en aval de la vanne de contrôle de pression et en dérivation de la ligne de soutirage.

10. Procédé de séparation d'au moins un isomère de xylène à partir d'une charge d'hydrocarbures en contenant, mis en oeuvre dans une unité de séparation selon l'une des revendications 1 à 8 **caractérisé en ce que** l'on établit une perte de charge appropriée en disposant au moins un organe de restriction (201 ) et de préférence plusieurs organes de restriction en parallèle ou en série, de taille adéquate, sur une ligne (60) de soutirage d'un fluide de la ou des colonnes entre au moins un des lits et une vanne (6) de contrôle de pression disposée sur ladite ligne de soutirage (extrait ou raffinat) de façon à obtenir une pression en amont de ladite vanne sensiblement constante ou dans une plage de valeurs comprises entre la valeur de pression de soutirage la plus faible PO et la dite valeur PO augmentée d'une fraction de la perte de charge de la colonne, et de préférence égale à au plus la dite valeur PO augmentée de 50% de la perte de charge de la colonne et de manière particulièrement préférée égale à au plus la dite valeur PO augmentée de 10% de la perte de charge de la colonne.

11. Procédé de séparation selon la revendication 10 **caractérisé en ce que** l'on établit une perte de charge appropriée en disposant au moins un organe de restriction et de préférence plusieurs organes de restriction, en parallèle ou en série, de taille adéquate, sur au moins une des lignes (130) d'injection d'un fluide (charge ou désorbant) dans la ou les colonnes entre la vanne de contrôle de débit disposée sur ladite ligne d'injection et entre au moins un des lits de façon à obtenir une pression en aval de ladite vanne sensiblement constante ou dans une plage de valeurs comprises entre la valeur de pression d'injection la plus forte PM et la dite valeur PM diminuée d'une fraction de la perte de charge de la colonne, et de préférence égale à au moins la dite valeur PM diminuée de 50% de la perte de charge de la colonne et de manière particulièrement préférée égale à au moins la dite valeur PM diminuée de 10% de la perte de charge de la colonne.

12. Procédé de séparation selon la revendication 10 dans lequel on régule la perte de charge aux bornes d'au moins une vanne de contrôle de débit disposée sur au moins une des lignes d'injection (charge ou désorbant) et dans lequel on place une vanne de régulation de pression différentielle en amont de la vanne de contrôle de débit, en dérivation de la ligne d'injection de fluide, de façon à obtenir une différence de pression aux bornes de la vanne de régulation de débit comprise entre une valeur de référence et la dite valeur de référence augmentée d'une fraction de la perte de charge de la colonne, et de préférence égale à au plus la dite valeur de référence augmentée de 50% de la perte de charge de la colonne et de manière particulièrement préférée égale à au plus la dite valeur de référence augmentée de 10% de la perte de charge de la colonne, la dite valeur de référence étant habituellement comprise entre 0,02 et 1 MPa et plus particulièrement entre 0,05 et 0,2 Mpa.

13. Procédé de séparation selon l'une des revendications 10 à 12 dans lequel on établit une perte de charge appropriée en disposant au moins un organe de restriction et de préférence plusieurs organes de restriction, en parallèle ou en série, de taille adéquate, sur une ligne de soutirage d'un fluide de la ou des colonnes entre au moins un des lits et une vanne de contrôle de débit disposée sur ladite ligne de soutirage (extrait ou raffinat) de façon à obtenir une pression en amont de ladite vanne sensiblement constante ou dans une plage de valeurs comprises entre la valeur de pression de soutirage la plus faible PO et la dite valeur PO augmentée d'une fraction de la perte de charge de la colonne, et de préférence égale à au plus la dite valeur PO augmentée de 50% de la perte de charge de la colonne et de manière particulièrement préférée égale à au plus la dite valeur PO augmentée de 10% de la perte de charge de la colonne.

14. Procédé de séparation selon l'une des revendications 10 à 12 dans lequel on régule la perte de charge aux bornes d'au moins une vanne de contrôle de débit ou de pression disposée sur l'un des soutirages (extrait ou raffinat) dans lequel on place une vanne de régulation de pression différentielle en aval de la vanne de contrôle de pression ou de débit, en dérivation de la ligne de soutirage de fluide, de façon à obtenir une différence de pression aux bornes de la dite vanne de régulation comprise entre une valeur de référence et la dite valeur de référence augmentée d'une fraction de la perte de charge de la colonne, et de préférence égale à au plus la dite valeur de référence augmentée de 50% de la perte de charge de la colonne et de manière particulièrement préférée égale à au plus à la dite valeur de référence augmentée de 10% de la perte de charge de la colonne, la dite valeur de référence étant habituellement comprise entre 0,02 et 1 MPa et plus particulièrement entre 0,05 et 0,2 Mpa.

## Claims

1. An installation for separating by simulated moving bed at least one xylene isomer from an hydrocarbon feedstock comprising such isomer, said installation comprising a plurality of beds disposed in a closed loop in at least one chromatographic column (1), at least one means for recirculating fluid (9) and comprising at least two lines for injecting fluid at a controlled flow rate and at least two lines for withdrawing fluid, one of said withdrawal (extract or raffinate) lines being under flow rate control, the other (60) being under pressure control via at least one control valve (6), **characterized in that** said pressure controlled withdrawal line (60) comprises at least one restriction means (201), and preferably several restriction means in parallel or in series enabling to reduce the pressure between one at least of said beds and the pressure control valve, that or these restriction means being constructed so as to keep upstream said valve a pressure which is appoximately constant or in the pressure range between the lowest withdrawal pressure PO and PO plus a fraction of the column pressure drop.

2. An Installation according to claim 1, in which said fraction of the column pressure drop is equal to 50% of said column pressure drop.

3. An Installation according to claim 2, in which said fraction of the column pressure drop is equal to 50% of said column pressure drop.

4. An installation according to any one of claims 1 to 3, in which the pressure controlled withdrawal line contains the raffinate.

5. An installation according to any one of claims 1 to 4, in which at least one of the injection lines (130) comprises a flow rate control valve (13) and at least one restriction means located between the (downstream) outlet from the flow rate control valve and at least one bed so as to maintain the pressure downstream of the flow rate control valve approximately constant or within the pressure range between the highest injection pressure PM and PM minus 50% of the column pressure drop.

6. An installation according to any one of claims 1 to 4, in which at least one of the injection lines comprises a flow rate control valve and at least one valve for regulating the pressure differential at the ports of the flow rate control valve, said differential pressure regulation valve being located upstream of the flow rate control valve and in parallel to the injection line.

7. An installation according to any one of claims 1 to 6, in which the other withdrawal line (120) comprises a flow rate control valve (12) and at least one restriction means located between at least one of the beds and the (upstream) inlet to the flow rate controlled withdrawal valve.

8. An installation according to any one of claims 1 to 6, in which the other withdrawal line comprises a flow rate control valve and at least one valve for regulating the pressure differential at the ports of the flow rate control valve, said differential pressure regulation valve being located downstream of the flow rate control valve and in parallel to the injection line.

9. An installation according to any one of claims 1 to 8, in which said withdrawal line which comprises a pressure control valve comprises, as the restriction means enabling the pressure to be reduced, at least one valve for regulating the pressure differential at the ports of the pressure control valve, said differential pressure regulation valve being located downstream of the pressure control valve and in parallel to the withdrawal line.

10. A process for separating at least one xylene isomer from an hydrocarbon feedstock comprising such isomer, carried out in an installation according to any one of claims 1 to 8, **characterized in that** a suitable pressure drop is established by providing at least one restriction means (201), preferably a plurality of restriction means in parallel or in series, of suitable size, in a line (60) for withdrawing fluid from the column or columns between at least one of the beds and a pressure control valve (6) located in said withdrawal line (extract or raffinate) so as to obtain a pressure upstream of said valve approximately constant or within a range of values in the range from the lowest withdrawal pressure P0 and said value P0 increased by a fraction of the pressure drop in the column, and preferably equal to at most said value P0 increased by 50% of the pressure drop in the column and particularly preferably equal to at most said value P0 increased by 10% of the pressure drop in the column.

11. A process according to claim 10, **characterized in that** a suitable pressure drop is established by providing at least one restriction means, preferably a plurality of restriction means in parallel or in series, of suitable size, in at least one of the lines for injecting fluid (feed or desorbent) into the column or columns between the flow rate control valve located in said injection line (feed or desorbent) and between at least one of said beds so as to obtain a pressure downstream of said valve approximately constant or within the range of values from the highest injection pressure PM and said value PM reduced by a fraction of the pressure drop in the column, preferably at least said value PM reduced by 50% of the pressure drop in the column and more particularly equal to at least said value PM reduced by 10% of the pressure drop in the column.

12. A process according to claim 10, in which the pressure drop at the ports of at least one flow rate control valve located in at least one of the injection lines (feed or desorbent) is regulated and in which a differential pressure regulation valve is placed upstream of the flow rate control valve, in parallel to the fluid injection line, so as to obtain a pressure difference at the ports of the flow rate regulation valve in the range from a reference value and said reference value increased by a fraction of the pressure drop in the column, preferably equal to at most said reference value increased by 50% of the pressure drop in the column, and particularly preferably equal to at most said reference value increased by 10% of the pressure drop in the column, said reference value usually being in the range 0.02 to 1 Mpa, more particularly in the range 0.05 to 0.2 Mpa.

13. A process according to any one of claims 10 to 12, in which a suitable pressure drop is established by providing at least one restriction means, preferably a plurality of restriction means in parallel or in series, of suitable size, in a line for withdrawing fluid from the column or columns between at least one of the beds and a flow rate control valve located in said withdrawal line (extract or raffinate) so as to obtain a pressure upstream of said valve approximately constant or within the range of values from the lowest withdrawal pressure P0 and said value P0 increased by a fraction of the pressure drop in the column, preferably equal to at most said value P0 increased by 50% of the pressure drop in the column and more particularly preferably equal to at most said value P0 increased by 10% of the pressure drop in the column.

14. A process according to any one of claims 10 to 12, in which the pressure drop at the ports of at least one flow rate or pressure control valve located in at least one of the injection lines (feed or desorbent) is regulated by placing a differential pressure regulation valve downstream of the flow rate control valve, in parallel to the fluid injection line, so as to obtain a pressure difference at the ports of the regulation valve in the range from a reference value and said reference value increased by a fraction of the pressure drop in the column, preferably equal to at most said reference value increased by 50% of the pressure drop in the column, and particularly preferably equal to at most said reference value increased by 10% of the pressure drop in the column, said reference value usually being in the range 0.02 to 1 Mpa, more particularly in the range 0.05 to 0.2 Mpa.

## Patentansprüche

1. Einheit zum Trennen, im simulierten beweglichen Bett, wenigstens eines Xylolisomers, ausgehend von einer es enthaltenden Kohlenwasserstoffcharge, mit einer Vielzahl von in geschlossener Schleife angeordneten Betten in wenigstens einer chromatographischen Kolonne (1), wenigstens ein Organ (9) zur Rezirkulation des Fluids, und mit wenigstens zwei Leitungen zum Infizieren von Fluid unter Mengenregelung und wenigstens zwei Fluidabzugsleitungen, wobei eine dieser Abzugsleitungen (Extrakt oder Raffinat) mengen- bzw. durchflussgeregelt ist, und die andere (60) vermittels wenigstens eines Regelventils (9) druckgeregelt ist,
**dadurch gekennzeichnet, dass** diese druckgeregelte Abzugsleitung (60) wenigstens ein Begrenzungsorgan (201) und bevorzugt mehrere Begrenzungsorgane, die parallel oder in Reihe angeordnet sind, umfasst, das oder die es ermöglicht bzw. ermöglichen, den Druck zwischen wenigstens den Betten und dem druckgeregelten Ventil zu senken, wobei das oder diese Organ(e) so ausgelegt sind, dass sie vor diesem Ventil einen im Wesentlichen konstanten Druck sicherstellen oder auch einen Druck im Inneren eines Bereichs von Werten, die zwischen dem geringsten Druckabzugswert PO und dem Wert PO sich befinden, der um einen Bruchteil des Druckverlustes der Kolonne erhöht ist.

2. Trenneinheit nach Anspruch 1, bei der diese Fraktion oder dieser Bruchteil des Druckverlustes der Kolonne 50 % des Druckverlustes der Kolonne ausmacht.

3. Trenneinheit nach Anspruch 2, bei der dieser Bruchteil des Druckverlustes der Kolonne 10 % des Druckverlustes der Kolonne ausmacht.

4. Trenneinheit nach einem der Ansprüche 1 bis 3, bei der die druckgeregelte Abzugsleitung das Raffinat enthält.

5. Trenneinheit nach einem der Ansprüche 1 bis 4, bei der wenigstens eine der Injektionsleitungen (130) ein Mengenregelventil (13) und wenigstens ein Begrenzungsorgan umfasst, das zwischen dem Austritt (stromabwärts) des Mengenregelventils und wenigstens einem Bett angeordnet ist, derart, dass der Druck hinter dem mengengeregelten Ventil im Wesentlichen konstant oder innerhalb eines Bereiches von Werten gehalten wird, die zwischen dem höchsten Wert der Druckeinspritzung PM und dem Wert PM liegt, der um 50 % des Chargendruckverlustes vermindert ist.

6. Trenneinheit nach einem der Ansprüche 1 bis 4, in der wenigstens eine der Einspritzleitungen ein druckgeregeltes Ventil und wenigstens ein Steuerventil für den Differentialdruck an den Klemmen des druckgeregelten Ventils umfasst, wobei dieses Differentialdrucksteuerventil vor dem mengengeregelten Ventil und parallel zur Einspritzleitung angeordnet ist.

7. Trenneinheit nach einem der Ansprüche 1 bis 6, in der die andere Abzugsleitung (120) ein mengengeregeltes Ventil (12) und wenigstens ein Begrenzungsorgan umfasst, das zwischen wenigstens einem der Betten und dem Eingang (anströmseitig) des mengengeregelten Abzugsventils angeordnet ist.

8. Trenneinheit nach einem der Ansprüche 1 bis 6, bei der die andere Abzugsleitung ein mengengeregeltes Ventil und wenigstens ein Steuerventil für den Differentialdruck an den Klemmen des mengengeregelten Ventils umfasst, wobei dieses Differentialdrucksteuerventil hinter dem mengengeregelten Ventil und parallel zur Abzugsleitung angeordnet ist.

9. Trenneinheit nach einem der Ansprüche 1 bis 8, bei der diese Abzugsleitung, die ein druckgeregeltes Ventil umfasst, als Begrenzungsorgan, das es ermöglicht, den Druck zu senken, wenigstens ein Steuerventil für den Differentialdruck an den Klemmen des druckgeregelten Ventils umfasst, wobei dieses Differentialdrucksteuerventil hinter dem mengengeregelten Ventil und parallel zur Abzugsleitung angeordnet ist.

10. Verfahren zur Trennung wenigstens eines Xylolisomers, ausgehend von einer es enthaltenden Kohlenwasserstoffcharge, verwirklicht in einer Trenneinheit gemäß einem der Ansprüche 1 bis 8, **dadurch gekennzeichnet, dass** man einen geeigneten Druckverlust herstellt, indem man wenigstens ein Begrenzungsorgan (201) und bevorzugt mehrere parallel oder in Reihe geschaltete Begrenzungsorgane geeigneter Abmessungen an einer Abzugsleitung (60) für ein Fluid der Kolonne(n) zwischen wenigstens einem der Betten und einem druckgeregelten Ventil (6) vorsieht, das an dieser Abzugsleitung (Extrakt oder Raffinat) derart angeordnet ist, dass man einen im Wesentlichen konstanten Druck vor diesem Ventil oder in einem Bereich von Werten zwischen dem geringsten Wert des Abzugsdrucks PO und dem Wert PO erhält, der um einen Bruchteil des Kolonnendruckverlustes erhöht ist und bevorzugt höchstens gleich diesem Wert PO, erhöht um 50 % des Druckverlustes der Kolonne, ist, und ganz besonders bevorzugt höchstens gleich diesem Wert PO, erhöht um 10 % des Kolonnendruckverlustes, ist.

11. Trennverfahren nach Anspruch 10, **dadurch gekennzeichnet, dass** man einen geeigneten Druckverlust einstellt, indem man wenigstens ein Begrenzungsorgan, bevorzugt mehrere Begrenzungsorgane, parallel oder in Reihe, geeigneter Abmessungen, an wenigstens einer der Injektionsleitungen (130) für ein Fluid (Charge oder Desorbens) in der oder den Kolonne(n) zwischen dem mengengeregelten Ventil, das an dieser Injektionsleitung sitzt und zwischen wenigstens einem der Betten angeordnet, derart, dass man einen im Wesentlichen konstanten Druck hinter diesem Ventil oder innerhalb eines Bereichs von Werten erhält, die zwischen dem höchsten Wert des Injektionsdrucks PM und diesem Wert PM, vermindert um einen Bruchteil des Druckverlustes der Kolonne und bevorzugt gleich wenigstens diesem Wert PM, vermindert um 50% des Druckverlustes der Kolonne und besonders bevorzugt wenigstens gleich diesem Wert PM, vermindert um 10 % des Kolonnendruckverlustes, erhält.

12. Trennverfahren nach Anspruch 10, bei dem man den Druckverlust an den Klemmen wenigstens eines mengengeregelten Ventils steuert, das an wenigstens einer der Injektionsleitungen (Charge oder Desorbens) angeordnet ist und bei dem man ein Differentialdrucksteuerventil vor dem mengengeregelten Ventil parallel zur Fluidinjektionsleitung derart anordnet, dass man eine Druckdifferenz an den Klemmen des mengengesteuerten Ventils zwischen einem Bezugswert und diesem Bezugswert, erhöht um einen Bruchteil des Druckverlustes der Kolonne und bevorzugt höchstens gleich dem Bezugswert, erhöht um 50 % des Kolonnendruckverlustes und besonders bevorzugt höchstens gleich diesem Bezugswert, erhöht um 10 % des Druckverlustes der Kolonne, erhält, wobei dieser Bezugswert üblicherweise zwischen 0,02 und 1 MPa und insbesondere zwischen 0,05 und 0,2 MPa liegt.

13. Trennverfahren nach einem der Ansprüche 10 bis 12, bei dem man einen geeigneten Druckverlust herstellt, indem man wenigstens ein Begrenzungsorgan und bevorzugt mehrere Begrenzungsorgane parallel oder in Reihe, geeigneter Abmessung, an einer Fluidabzugsleitung der Kolonne(n) zwischen einem der Betten und einem mengengeregelten Ventil anordnet, das an dieser Abzugsleitung (Extrakt oder Raffinat) sitzt, derart, dass man einen im Wesentlichen konstanten Druck vor diesem Ventil oder einen Druck in einem Bereich von Werten erhält, die zwischen dem geringsten Wert des Abzugsdrucks PO und diesem Wert PO, erhöht um einen Bruchteil des Kolonnendruckverlustes, erhält und bevorzugt höchstens gleich diesem Wert PO, erhöht um 50 % des Kolonnendruckverlustes und besonders bevorzugt höchstens gleich diesem Wert PO, erhöht um 10 % des Kolonnendruckverlustes, erhält.

14. Trennverfahren nach einem der Ansprüche 10 bis 12, bei dem man den Druckverlust an den Klemmen wenigstens eines mengen- oder druckgeregelten Ventils erhält, das an einer der Abzugsleitungen (Extrakt oder Raffinat) angeordnet ist, bei dem man ein Ventil zur Steuerung des Differentialdrucks hinter dem druck- oder mengengeregelten Ventil parallel zur Abzugsleitung des Fluids anordnet, derart, dass man eine Druckdifferenz an den Klemmen dieses Steuerventils zwischen einem Bezugswert und dem Bezugswert, erhöht um einen Bruchteil des Druckverlustes der Kolonne und bevorzugt höchstens gleich diesem Bezugswert, erhöht um 50 % des Kolonnendruckverlustes und besonders bevorzugt höchstens gleich diesem Bezugswert, erhöht um 10 % des Druckverlustes der Kolonne, erhält, wobei dieser Bezugswert üblicherweise zwischen 0,02 und 1 MPa und insbesondere zwischen 0,05 und 0,2 MPa beträgt.
